Europäisches Patentamt

European Patent Office

Office européen des brevets

(19)

(11) Veröffentlichungsnummer: **0 135 060**
**B1**

(12) # EUROPÄISCHE PATENTSCHRIFT

(45) Veröffentlichungstag der Patentschrift:
03.05.89

(21) Anmeldenummer: **84108763.8**

(22) Anmeldetag: **25.07.84**

(51) Int. Cl.⁴: **A 61 K 7/035,** A 61 K 9/20

(54) Feste Puder-Zubereitung und Verfahren zur Herstellung derselben.

(30) Priorität: **27.07.83 DE 3327001**

(43) Veröffentlichungstag der Anmeldung:
**27.03.85 Patentblatt 85/13**

(45) Bekanntmachung des Hinweises auf die Patenterteilung:
**03.05.89 Patentblatt 89/18**

(84) Benannte Vertragsstaaten:
**AT BE CH DE FR GB IT LI LU NL SE**

(56) Entgegenhaltungen:
**GB-A- 1 141 994**
**GB-A- 1 357 731**

**CHEMICAL ABSTRACTS, Band 98, Nr. 22, May 1983,**
**Seite 360, Nr. 185407c, Columbus, Ohio, US; & JP - A - 58**
**39 609 (SHISEIDO CO. LTD.) 08.03.1983**
**CHEMICAL ABSTRACTS, Band 101, Nr. 10, September**
**1984, Seite 368, Nr. 78684z, Columbus, Ohio, US; & JP -**
**A - 59 93 013 (POLA CHEMICAL INDUSTRIES INC)**
**29.05.1984**

(73) Patentinhaber: **Württembergische Parfümerie-Fabrik**
**GmbH, Schillerstrasse 21-27,**
**D-7332 Eislingen/Fils 16 (DE)**

(72) Erfinder: **Scheller, Hans-Ulrich, Dr.,**
**Vogelgartenstrasse 45, D-7332 Eislingen/Fils (DE)**
Erfinder: ** Köddermann, Else, Mühlweg 5,**
**D-7333 Ebersbach/Fils (DE)**

(74) Vertreter: **Werner, Hans-Karsten, Dr. et al,**
**Deichmannhaus am Hauptbahnhof, D-5000 Köln 1 (DE)**

**Beschreibung**

Gegenstand der vorliegenden Erfindung sind feste Puder-Zubereitungen für kosmetische und medizinische Zwecke, welche einen oder mehrere Füllstoffe, einen oder mehrere fettartige und wachsartige Bestandteile, einen oder mehrere Wirkstoffe und/oder Farbstoffe, ein oder mehrere verdampfbare, untoxische, hydrophobe Lösungsmittel, sowie ggf. weitere Zusätze enthalten, sowie Verfahren zur Herstellung derselben.

Mit Pigmenten angefärbte Puder werden seit Jahrhunderten zu kosmetischen Zwecken, nämlich zur Verbesserung des Hautbildes und zum Hervorheben des jeweils gültigen Schönheitsideals verwendet. Diese Puder wurden ursprünglich als lose Puder, später auch als Puderkreiden hergestellt. Die Pudergrundlagen waren vorwiegend Füllstoffe, wie Talkum, Kaolin, Kreiden von unterschiedlicher Feinheit, Erdalkalicarbonate, ungefärbte Oxide des Aluminiums, Titans, Zinks, Metallseifen wie Aluminium-, Magnesium-, und Zinkstearate oder Myristate. Als Binder dienten die verschiedensten Arten von Stärken, aber auch Seidenpulver und Zellulosen.

Aus «Chemical Abstracts» 98, 185407 c und JP-A-5 839 609 sind kosmetische Puder bekannt, die hydrophobes $SiO_2$ neben Wasser und synthetischem Polymerpulver (z.B. Polymid) enthalten.

Die GB-A-1 141 994 beschreibt eine kosmetische Zubereitung zur Anwendung auf der menschlichen Haut, in denen 5 bis 95 Gew.-% der pulverförmigen Teilchen Mikrokugeln aus Polymerisaten sind mit einem durchschnittlichen Durchmesser zwischen 1 und 100 Mikrometer. Insbesondere handelt es sich um Mikrokugeln aus quervernetzten Polymeren wie Styrol, Divinylbenzol sowie gegebenenfalls Metallseifen, wie Magnesiumstearat.

Die GB-A-1 357 731 offenbart freifließende Puder-Zubereitungen, die Stärke enthalten und deren Teilchengröße nicht größer als 60 Mikrometer sein sollte.

Pudersteine wurden dadurch hergestellt, daß man die Farbpigmente und Füllstoffe mit einem Binder, wie Calciumsulfat, mit Wasser anrührte, in flache Pfannen goß und an der Luft trocknen ließ. Die so erhaltenen Puder wurden mit einem feuchten Schwamm aufgetragen. Derartige Pudersteine erfüllen in keiner Weise die heute an Schminkmittel gestellten Anforderungen und werden daher seit langem nicht mehr hergestellt.

Seit etwa 30 Jahren verwendet man fast ausschließlich sogenannte Kompakt-Puder, die man durch Kompression von losen Puderpulvern in Metallpfannen (auch Tabletts genannt) bei Preß-Drücken zwischen 20 und 50 bar herstellt. Der aufzuwendende Preß-Druck hängt dabei individuell von der zu pressenden Puder-Rezeptur ab, beispielsweise von den im Binder verwendeten Hydrokolloiden oder niedrigviskosen Carboxymethylzellulosen sowie deren Feuchtigkeitsgrad und dem Anteil und Preßverhalten der Farb- und Füll-Pigmente.

Bei der Herstellung dieser Kompakt-Puder ist ein Mindestdruck erforderlich, um bruchfeste Pudertabletts zu erhalten. Zu starkes Pressen führt jedoch zu harten und zu kompakten Produkten, die kaum mehr Puder abgeben. Zu geringe Preß-Drucke setzen dagegen die Transportfähigkeit der Tabletts bis zur Unbrauchbarkeit herab. Nach dem Pressen muß noch ein Sterilisationsprozeß angeschlossen werden. Die gepreßten Pfannen müssen zum Transport sehr sorgfältig verpackt werden, um sie ohne mechanische Beschädigung der empfindlichen Oberflächen manuell in die endgültigen Verpackungsboxen einsetzen zu können. Aus all diesem ergeben sich für die Herstellung von einwandfreien Gebrauchsqualitäten sehr hohe Ausschußquoten und damit letztlich sehr hohe Entstehungskosten.

Aus der CA-PS 1 125 659 ist ein Verfahren bekannt, kompakte Puder-Zubereitungen dadurch herzustellen, daß man bei erhöhten Temperaturen Fettalkohole mit 12 bis 20 Kohlenstoffatomen, einen feinverteilten Füllstoff und ein verdampfbares Siloxan zu einer fließfähigen Masse verrührt, in die gewünschten Formen gießt und anschließend den Überschuß des Siloxans verdampft. Die so erhaltenen Produkte wiesen zwar gegenüber herkömmlichen Wachsstiften Vorteile auf und ließen sich verfahrensmäßig leichter herstellen, jedoch war ihr äußeres Aussehen so, daß sie den heutigen Anforderungen in keiner Weise genügten. Beim Verdampfen des Lösungsmittels führten diese Produkte nämlich zu unschönen Rissen und Abblätterungen, die vom Verbraucher keinesfalls akzeptiert worden wären.

Für die Herstellung kosmetischer Artikel wurde daher ein Verfahren entwickelt, bei welchem die fließfähigen Massen gemäß CA-PS 1 125 659 umgekehrt in Formen gegossen wurden, die an der Unterseite ein Gitter aufwiesen und bei denen die Verdampfung des Lösungsmittels durch die Unterseite erfolgen konnte; vgl. US-PS 4 337 859. Die unerwünschte Wanderung der Fettalkohole beim Verdampfen des Lösungsmittels führten an der Oberfläche zu einer Verhärtung des Puderkuchens, die an sich unerwünscht ist. Bei dem Verfahren gemäß US-PS 4 337 859 wird dieser Effekt dazu ausgenutzt, die fertige Zubereitung fester mit dem Gitter am Boden zu verbinden.

Es leuchtet ein, daß weder die Gemische gemäß CA-PS 1 125 659 noch das Verfahren zu ihrer Verarbeitung gemäß US-PS 4 337 859 in der Lage waren, die bisher verwendeten durch Pulverpressung erzeugten Tabletts zu verdrängen.

Die Erfindung hat sich die Aufgabe gestellt, feste Puder-Zubereitungen zu entwickeln, welche in ähnlicher Weise wie die gegossenen Puder-Zubereitungen gemäß CA-PS 1 125 659 herstellbar sind, jedoch leichter zu verarbeiten sind und unmittelbar zu Produkten führen, die mit der Qualität herkömmlicher Kompakt-Puder vergleichbar sind.

Diese Aufgabe kann überraschenderweise gelöst werden durch eine kompakte Puder-Zubereitung enthaltend

a) einen oder mehrere Füllstoffe,

b) einen oder mehrere fettartige und wachsartige Bestandteile,

c) einen oder mehrere Wirkstoffe und/oder Farbstoffe,

d) ein oder mehrere verdampfbare, untoxische,
hydrophobe Lösungsmittel und

e) ggf. weitere übliche Zusätze,

dadurch gekennzeichnet, daß der oder die Füllstoffe nicht hygroskopische, rieselfähige, organische oder anorganische Feststoffe sind mit einer
Teilchengröße von 5 bis 50 µm Durchmesser.

Diese Ergebnisse waren nicht vorherzusehen,
da der Fachmann bisher von der Annahme ausging, daß alle Puder zur Erzielung eines angenehmen weichen Puderaufstrichs die Mitverwendung
von Silikaten mit plattig-kristalliner Struktur wie
Talkum, Glimmer etc. erfordern. Es wurde jetzt
gefunden, daß hydrophobe, rieselfähige, organische oder anorganische Feststoffe mit einer Teilchengröße von 5 bis 50 µm Durchmesser ebenfalls
geeignet sind, einen angenehmen weichen Puderaufstrich zu gewährleisten und darüberhinaus die
Verdampfung des Lösungsmittels gestatten, ohne
daß sich bei diesem Vorgang die Masse an der
Oberfläche aufbläht und abbröckelt. Die erfindungsgemäße feste Puder-Zubereitung kann daher dadurch hergestellt werden, daß man das Gemisch aus den Bestandteilen a) bis e) mit einem
Überschuß an Lösungsmitteln bei Temperaturen
von über 40°C zu einer fließfähigen Masse verrührt, in die gewünschten Formen gießt, den Überschuß des Lösungsmittels verdampft und gewünschtenfalls die Oberfläche der so erhaltenen
Produkte mit Drücken unter 10 bar bearbeitet.

Erstaunlicherweise ist die Oberfläche der so
hergestellten kompakten Puder-Zubereitungen
glatt und einheitlich. Die Oberfläche kann jedoch
gewünschtenfalls bei Anwendung geringer Drükke, vorzugsweise von 3 bis 7 bar mit einem Dekorationsmuster versehen werden. Derartige Dekorationsmuster gestatten die einfache Überprüfung, ob die Puder-Zubereitung schon benutzt
wurde oder noch unversehrt ist. Diese geringen
Drucke, die auch nur auf die Oberfläche ausgeübt
werden, werden auch von den üblichen Formen
ohne Beschädigung vertragen. Erfindungsgemäß
können die Formen nahezu beliebig variiert werden, so daß man nicht mehr an die genormten
Tabletts gebunden ist.

Als nicht-hygroskopische, rieselfähige, organische oder anorganische Feststoffe können prinzipiell alle Pulver aus Polyethylen, Polypropylen,
Polyvinylpyrrolidon, Polyamid, aber auch hydrophoben Salzen, wie Tricalciumphosphat, verwendet werden. Diese Produkte sind insbesondere
dann rieselfähig, wenn sie aus kugelförmigen
oder nahezu kugelförmigen Teilchen bestehen.
Besonders geeignet sind solche Pulver, die überwiegend aus einer relativ engen Siebfraktion
stammen oder aufgrund ihres Herstellungsverfahrens eine möglichst enge Größenverteilungskurve
aufweisen. Derartige Pulver neigen nicht zum Agglomerieren und gewährleisten eine besonders
gute und einwandfreie Verarbeitbarkeit, verbunden mit sehr guten Eigenschaften des Endproduktes.

Bevorzugt werden daher Pulver, die mindestens zu 70% Korngrößen von 10 bis 25 µm Durchmesser aufweisen.

Die Menge aller erfindungsgemäß verwendeten
Füllstoffe ist nicht kritisch. Es können Mengen von
10 bis 80 Gew.-% eingesetzt werden. Die optimale
Menge hängt jedoch von Art und Menge der übrigen Bestandteile ab. Das gleiche gilt für die fettartigen und wachsartigen Bestandteile, die einerseits zu einer ausreichenden Verklebung der kompakten Puder-Zubereitung führen soll, jedoch so
auszuwählen sind, daß der auf der Haut aufgetragene Puder möglichst wenig schmiert oder wandert. Die fettartigen und wachsartigen Bestandteile sollten weiterhin so gewählt sein, daß sie zumindest mit Detergentien von der Haut und aus
der Wäsche entfernt werden können. Für kosmetische Zubereitungen hat es sich als vorteilhaft erwiesen, die Wirkstoffe oder Farbstoffe mit flüssigen oder niedrig schmelzenden Wachsen oder
Wachsestern zu Pasten zu vermahlen, die sich
dann ohne Schwierigkeiten in der gießfähigen
Masse dispergieren lassen. Der Anteil an derartigen Komponenten liegt im allgemeinen zwischen
1 und 20 Gew.-%, vorzugsweise 5 bis 10 Gew.-%
der gießfähigen Masse.

Als weitere Bestandteile dieser Phase haben
sich Glycerin-mono-dioleat und Fettsäure-monoethanol-amide, wie Stearinsäure-mono-ethanolamid, besonders bewährt, da sie zu einer guten
Haftung der Pigmente auf der Haut führen. Sie
werden in Mengen von 0,1 bis 10 Gew.-%, vorzugsweise 0,1 bis 5 Gew.-% der gießfähigen Masse verwendet. Prinzipiell lassen sich aber auch für
alle sonstigen kosmetischen und medizinischen
Zwecke geeignete Fette und Wachse für die erfindungsgemäßen Puder-Zubereitungen verwenden.

Als fettartige und wachsartige Bestandteile können Fette, flüssige Fette, Wachse und ähnliche
natürliche und/oder synthetische Substanzen angewandt werden. Diese Gruppe umfaßt nicht nur
die echten Fette, d.h. die Triglyceride der höheren
Fettsäuren sondern auch die verschiedenen flüssigen und festen Wachse, die verschiedenen Fettsäureester, die Fettalkohole, die Ester und insbesondere die polyvalenten Alkohole, die Polyethylenglykole und ihre Derivate, Paraffinöle, Petrolatum (Vaseline[ⓡ]), Parraffine und Silikonöle. Es
kommen alle Substanzen in Betracht, welche unter bestimmten Bedingungen und insbesondere in
geeigneten Konzentrationen hautpflegend wirken,
wie die bei Raumtemperatur flüssigen oder festen
echten Fette, und welche nach ihrer Anwendung
die Haut einfetten ohne einen sichtbaren Film auf
der Haut zu hinterlassen. Solche Substanzen sind
bekannt und werden in allgemein verfügbaren
Handbüchern genau beschrieben, z.B. H.P. Fiedler, Lexikon der Hilfsstoffe für Pharmazie, Kosmetik und andere angrenzende Gebiete, Herausgeber Cantor Verlag, Aulendorf, Württemberg 1971,
S. 14 bis 16; H. Janistyn, Handbuch der Kosmetika
und Riechstoffe, 1. Band: die kosmetischen Grundstoffe, Dr. Alfred Huethig Verlag, Heidelberg, 1969,
S. 378 bis 399 und G.A. Nowak, die kosmetischen
Präparate, 2. Auflage, Verlag für chemische Industrie, H. Ziolowsky, Augsburg, 1975 S. 192 bis 198, 218 ff. 309 bis 312 und 424 bis 449.

Als Wirkstoffe können geruchsverhindernde Substanzen, Lichtschutzmittel, Desinfektionsmittel und sonstige auf der Haut oder in der Haut wirkenden Substanzen eingesetzt werden, insbesondere wenn es sich um feuchtigkeitsempfindliche Substanzen handelt. Als Farbstoffe werden vorzugsweise Farbpigmente verwendet, obwohl es prinzipiell auch möglich ist, für bestimmte Anwendungen wasserlösliche oder fettlösliche Farbstoffe einzusetzen. Die Mengen an Farbpigmenten können im Extremfall zwischen 2 und 80 Gew.-% der kompakten Puder-Zubereitung ausmachen. Im allgemeinen werden Mengen zwischen 30 und 60% eingesetzt. Bei sehr hohem Anteil an Farbpigmenten ist darauf zu achten, daß die Teilchenform und Teilchengröße nicht wesentlich von der der Füllstoffe abweicht.

Als verdampfbare, untoxische hydrophobe Lösungsmittel können prinzipiell alle bekannten Lösungsmittel eingesetzt werden. Vorzugsweise werden solche Lösungsmittel verwendet, deren Verdunstungszahl kleiner als 80 ist, insbesondere Kohlenwasserstoffe wie n-Pentane, Isopentane, n-Hexane, n-Heptane, Isoparaffine, Cycloalkane, Kohlenwasserstoffe, die mit Chlor und/oder Fluor substituiert sind, Alkohole wie Ethanol, n-Propanol, Isopropanol, n-Butanol, Ethylenglykol sowie Äther, Ketone, Ester wie Essigsäure, Ethyläther und Ethylenglykolmonoacetat. Besonders bewährt haben sich Silikonöle, insbesondere die cyclischen Silikonöle mit 4 oder 5 Siliciumatomen. Von diesen Lösungsmitteln verbleiben in der festen Zubereitung auch nach dem Verdampfen 2 bis 5 Gew.-%. Zur Herstellung der gießfähigen und fließfähigen Masse werden im allgemeinen Mengen von 20 bis 60 Gew.-% in der Masse eingesetzt. Aus Gründen des Umweltschutzes sowie aus Kostengründen werden die verdampften Mengen des Lösungsmittels vorzugsweise wieder aufgefangen und erneut verwendet. Dies kann bei den Silikonölen besonders einfach dadurch erfolgen, daß man die Trockenluft nach dem Abkühlen durch Waschflaschen leitet, die mit den Lösungsmitteln gefüllt sind. Die verbleibenden 2 bis 5% Silikonöl in der festen Puder-Zubereitung sind durchaus erwünscht, da sie dem Puder zusätzlich gute Verreibbarkeit vermitteln und den Puder-Aufstrich auf der Haut transpirations- und regenfester macht.

Cyclische Silikonöle, wie Polydimethylcyclosiloxan, kann man bei Temperaturen zwischen 45 und 85 °C einwandfrei verarbeiten.

Als weitere übliche Zusätze können die erfindungsgemäßen kompakten Puder-Zubereitungen beispielsweise Antioxidantien, Konservierungsmittel und Parfums zugesetzt werden. Sofern insbesondere Parfums bei der Verdampfung des Lösungsmittels mit entweichen, müssen sie der fließfähigen Masse in entsprechend erhöhter Menge zugesetzt werden.

Zur Durchführung des erfindungsgemäßen Verfahrens werden die einzelnen Bestandteile bei Temperaturen über 40 °C, vorzugsweise bei Temperaturen zwischen 75 und 90 °C, in einem heizbaren Kessel miteinander vermischt und homogenisiert. Die so erhaltene fließfähige Masse wird vorzugsweise mit Hilfe eines Gießkessels mit Portionierautomatik in die gewünschten Formen gegossen. Für den Fall, daß das Gießen erst zu einem späteren Zeitpunkt stattfinden soll, ist es durchaus möglich, die Masse in verschlossenen Gefäßen bei Zimmertemperatur zu lagern und vor der Verarbeitung erneut aufzuschmelzen.

Beim Gießen der gießfähigen Masse in die Formen ist darauf zu achten, daß die Gießmasse beim Einlaufen nicht unter 45 °C auskühlt. Deshalb sind die Formen erforderlichenfalls beispielsweise mit Hilfe von trockener Warmluft vorzuwärmen.

Die erfindungsgemäßen Puder-Zubereitungen neigen weder beim Abkühlen noch beim Verdampfen des Lösungsmittels zum Schrumpfen. Sie haften daher ohne weiteres in den Gießformen. Um eine besonders feste Haftung zu gewährleisten, ist es jedoch möglich, den Rand und den Boden der Formen aufzurauhen oder zu strukturieren.

Die gefüllten Formen werden nach dem Abkühlen und Stocken der flüssigen Masse (unter 40 °C), vorzugsweise in Trockenschränken mit erwärmter Umluft bei 50 bis 60 °C gelagert, wobei der überwiegende Teil des überschüssigen Lösungsmittels verdampft. Das überschüssige Lösungsmittel in der Umluft kann in einfacher Weise wiedergewonnen und wiederverwendet werden. Bei Verwendung von Silikonöl reichen beispielsweise nachgeschaltete Waschkolonnen, die mit Silikonöl gefüllt sind.

Die Trocknung ist beendet, sobald sich die Puder-Zubereitungen ausreichend verfestigt haben, jedoch die Puderoberfläche sich noch immer mittels Applikator oder mit der trockenen Fingerkuppe seidenweich und hauchdünn verstreichbar abnehmen läßt. Vorzugsweise beläßt man 2 bis 3% des Lösungsmittels, maximal jedoch 5% des Lösungsmittels in der kompakten Zubereitung. Insbesondere bei Silikonöl führt dies zu besserer Verreibbarkeit und erhöht die Transpirations- und Regenfestigkeit des Farbpuderaufstriches auf der Haut.

Erforderlichenfalls können die kompakten Zubereitungen in den Formen visuell auf etwaige Gießfehler überprüft werden. Die Ausschußquote ist jedoch erstaunlich gering. Gewünschtenfalls wird die Oberfläche nachträglich mittels eines sanften pneumatisch betriebenen Stempels mit Drucken von ca. 5 bar strukturiert oder gekennzeichnet.

Sofern die Formen nicht schon vor dem Befüllen etikettiert und codiert waren, kann dies auch nach dem Füllen erfolgen.

In den nachfolgenden Beispielen werden typische kompakte Puder-Zubereitungen und Verfahren zu ihrer Herstellung näher erläutert.

Beispiel 1

Eine fließfähige Masse zur Herstellung fester Puder-Zubereitungen hat folgende Zusammensetzung:

| Oleylerucat | 7,20 Gew.-% |
|---|---|
| Stearinsäure-mono-ethanolamid | 1,00 Gew.-% |
| Glyceryloleat | 2,00 Gew.-% |
| Methylparaben | 0,20 Gew.-% |
| Propylparaben | 0,10 Gew.-% |
| Buthylhydroxytoluol (Antioxidans) | 0,10 Gew.-% |
| Polyamid (Nylon-12)-Pulver | 12,00 Gew.-% |
| Polydimethylsiloxan, chemische Bezeichnung Octamethylcyclotetrasiloxan (Cyclomethicone) | 48,70 Gew.-% |
| Methylchlorisothiazolinon + Methylisothiazolinon | 0,20 Gew.-% |
| Pigmente + Pearl-Pigmente | 28,50 Gew.-% |
| | 100,00 Gew.-% |

Das Polyamid (Nylon-12)-Pulver besteht aus überwiegend kugelförmigen und ellipsoiden Körpern mit einem Schüttgewicht von 0,20 bis 0,35 und einer Dichte von 1,02 bis 1,03. Die Korngrößenverteilung beträgt:

| über 40 μm | Korngrößen-Anteil unter | 0,5% |
|---|---|---|
| 40–30 μm | Korngrößen-Anteil unter | 6,0% |
| 30–20 μm | Korngrößen-Anteil unter | 10,0% |
| 20–10 μm | Korngrößen-Anteil über | 40,0% |
| 10– 5 μm | Korngrößen-Anteil über | 30,0% |

Unter Berücksichtigung der Standardabweichung von 3,51 μm ergibt sich eine durchschnittliche Korngröße für das Pulver von 12,1 μm. Das Pulver ist hydrophob und nimmt maximal 1% Luftfeuchtigkeit auf.

Ein Teil des Wachses und Fettes wird dazu benutzt, die Pigmente zu einer homogenen Farbpaste zu verrühren. Der übrige Anteil wird in einen Doppelwandkessel mit Rührwerk und Dispergator eingegeben. Weiterhin gibt man das Antioxidans und die Konservierungsmittel in den Kessel und schmilzt unter Rühren bei 85 °C auf. Jetzt werden die Farbpasten aus Pigmenten und einem Teil der Wachs- und Fettphasen homogen eingerührt. Die Temperatur wird auf 80 bis 85 °C gehalten. Jetzt wird das auf Zimmertemperatur belassene Silikonöl zusammen mit dem temperaturempfindlichen Konservierungsmittel Methylchlorisothiazolinon + Methylisothiazolinon zugefügt und gleichmäßig untergerührt. Als nächstes werden das Polyamid (Nylon-12)-Pulver und schließlich die trokkenen Pearl-Pigmente pulvrig eingerührt. Während des Homogenisierens darf die Temperatur auf 75 °C abfallen. Bei sorgfältiger Arbeit ist ein Entlüften der Gießmasse nicht notwendig. Sollten durch unvorsichtiges Arbeiten Lufteinschlüsse erkennbar sein, können diese durch leichten Unterdruck von maximal 0,5 bar entfernt werden. Die Gießmasse ist jetzt fertig und wird bei 75 °C in einen Gießkessel mit Portionierautomatik zum Befüllen von Puderboxen abgelassen. In Abhängigkeit von der Größe und der Dimension der Boxen kann es erforderlich sein, diese vorher mit trockener Warmluft auf etwa 30 °C zu temperieren, so daß beim Einlaufen der Gießmasse die Temperatur nicht unter 45 °C sinkt. Nach ca. 1 bis 2 Minuten und Abkühlen auf eine Temperatur unter 45 °C erstarrt die gegossene Masse zu einer halbfesten Creme. Die gefüllten Formen werden jetzt in einen mit starker Umluft durchflossenen Trockenschrank bei 60 °C schichtweise eingelagert und hierin etwa 15 Stunden belassen. Der stetig durchfließende Luftstrom nimmt das langsam abdampfende flüchtige Silikonöl mit. Es wird durch nachgeschaltete und kühlgehaltene Waschkolonnen, die mit dem gleichen Silikonöl gefüllt sind, aus der Umluft entfernt und kann erneut eingesetzt werden.

Die Trocknung ist beendet, sobald sich die Pudermassen vollständig verkompaktiert haben, jedoch sich noch mittels Applikator oder der trockenen Fingerkuppe seidenweich und hauchdünn verstreichbar abnehmen lassen. Der Gehalt an Silikonöl beträgt maximal 5%, vorzugsweise 2 bis 3%.

Die Oberfläche der gegossenen Formen läßt sich mit einem Stempel und einem Druck von ca. 5 bar signieren oder mit einem Strukturmuster versehen.

Bei Zusatz von 0,20 bis 0,50 Gew.-% Parfum wird die Menge des Silikonöls entsprechend verringert.

### Beispiele 2 und 3

In analoger Weise wie in Beispiel 1 beschrieben, wurden folgende Komponenten miteinander zu einer kompakten Puder-Zubereitung verarbeitet.

| | Beispiel 2 Gew.-% | Beispiel 3 Gew.-% |
|---|---|---|
| Oleylerucat | 7,20 | 7,20 |
| Stearinsäure-mono-ethanolamid | 1,00 | 1,00 |
| Glyceryloleat | 2,00 | 2,00 |
| Methylparaben | 0,20 | 0,20 |
| Propylparaben | 0,10 | 0,10 |
| Butylhydroxytoluol (Antioxidans) | 0,10 | 0,10 |
| Tricalciumphosphat | 12,00 | –.— |
| Polyethylen | –.— | 15,00 |
| Cyclomethicone (s. Beispiel 1) | 56,20 | 53,20 |
| Methylchlorisothiazolinon + Methylisothiazolinon | 0,20 | 0,20 |
| Pigmente + Pearl-Pigmente | 21,00 | 21,00 |
| | 100,00 | 100,00 |

Der durchschnittliche Teilchendurchmesser des Tricalciumphosphats betrug 6 bis 8 µm. Der durchschnittliche Durchmesser des Polyethylenpulvers betrug 6 µm. Bei Zusatz von Parfums wurde der Anteil des Silikonöls entsprechend reduziert.

Die erhaltenen Produkte hatten ebenfalls ein gutes Aussehen und ließen sich mit einem Applikator oder der trockenen Fingerkuppe seidenweich und hauchdünn zerstreichbar abnehmen. Es wurden keine Aufschäumungen oder Abblätterungen beobachtet wie bei Rezepturen gemäß CA-PS 1 125 659.

**Patentansprüche**

1. Kompakte Puder-Zubereitung, enthaltend
a) einen oder mehrere Füllstoffe,
b) einen oder mehrere fettartige und wachsartige Bestandteile,
c) einen oder mehrere Wirkstoffe und/oder Farbstoffe,
d) ein oder mehrere verdampfbare, untoxische, hydrophobe Lösungsmittel,
e) ggf. weitere übliche Zusätze,
dadurch gekennzeichnet, daß der oder die Füllstoffe nicht hygroskopische, rieselfähige, organische oder anorganische Feststoffe sind mit einer Teilchengröße von 5 bis 50 µm Durchmesser.

2. Zubereitung gemäß Anspruch 1, dadurch gekennzeichnet, daß die Füllstoffe kugelförmige oder ellipsoidförmige Pulver aus Polyethylen, Polypropylen, Polyvinylpyrrolidon, Polyamid oder hydrophoben anorganischen Salzen sind.

3. Zubereitung gemäß Anspruch 1 oder 2, dadurch gekennzeichnet, daß mindestens 70% des Füllstoffes eine Teilchengröße von 10 bis 25 µm aufweist.

4. Verfahren zur Herstellung von kompakten Puder-Zubereitungen gemäß einem der Ansprüche 1 bis 3, dadurch gekennzeichnet, daß man das Gemisch mit einem Überschuß an Lösungsmittel d) bei Temperaturen von über 40 °C zu einer fließfähigen Masse verrührt, in die gewünschten Formen gießt, den Überschuß des Lösungsmittels verdampft und gewünschtenfalls die Oberfläche der so erhaltenen Produkte mit Drücken unter 10 bar bearbeitet.

5. Verfahren gemäß Anspruch 4, dadurch gekennzeichnet, daß das Lösungsmittel ein Silikonöl ist.

6. Verfahren gemäß Ansprüchen 4 oder 5, dadurch gekennzeichnet, daß der verdampfte Überschuß des Lösungsmittels aufgefangen und wiederverwendet wird.

**Claims**

1. A compact powder formulation, containing
a) one or more fillers,
b) one or more fatlike and waxy components,
c) one or more active ingredients and/or dyes,
d) one or more evaporable non-toxic hydrophobic solvents,
e) optionally further conventional additives,
characterized in that the filler(s) is/are non-hygroscopic flowable organic or inorganic solids having a particle size from 5 to 50 µm in diameter.

2. The formulation according to claim 1, characterized in that the fillers are spherical or ellipsoidal powders of polyethylene, polypropylene, polyvinylpyrrolidone, polyamide or hydrophobic inorganic salts.

3. The formulation according to claim 1 or 2, characterized in that at least 70% of the filler have a particle size from 10 to 25 µm.

4. A process for preparing compact powder formulations according to any one of claims 1 to 3, characterized in that the mixture is stirred with an excess of solvent d) at a temperature in excess of 40 °C to form a flowable mass, said mass is cast into the desired shape, the excess of solvent is evaporated and, if desired, the surface of the products thus obtained is subjected to the action of pressures of below 10 bar.

5. The process according to claim 4, characterized in that the solvent is a silicone oil.

6. The process according to claims 4 or 5, characterized in that the evaporated excess of the solvent is collected and re-used.

**Revendications**

1. Préparation compacte de poudre contenant
a) une ou plusieurs matières de charges,
b) un ou plusieurs composants céreux et gras,
c) un ou plusieurs agents actifs et/ou colorants,
d) un ou plusieurs solvants évaporables, non toxiques, hydrophobes,
e) éventuellement d'autres additifs habituels,
caractérisée en ce que la ou les matières de charge sont des solides, non hydroscopiques, capables de s'écouler, organiques ou inorganiques avec une dimension de particules de 5 à 50 µm de diamètre.

2. Préparation selon la revendication 1, caractérisée en ce que les matières de charge sont des poudres sphériques ou ellipsoïdales de polyéthylène, de polypropylène, de polyvinylpyrrolidone, de polyamides ou de sels inorganiques hydrophobes.

3. Préparation selon la revendication 1 ou 2, caractérisée en ce qu'au moins 70% de la matière de charge présente une dimension de particules de 10 à 25 µm.

4. Procédé de production de préparations compactes de poudres selon l'une des revendications 1 à 3, caractérisé en ce qu'on brasse le mélange avec un excès de solvant d) à des températures supérieures à 40 °C en une masse coulante, en ce qu'on la moule à la forme souhaitée, en ce qu'on évapore l'excès de solvant, et en ce que, si on le souhaite, on traite la surface des produits ainsi obtenus sous des pressions inférieures à 10 bars.

5. Procédé selon la revendication 4, caractérisé en ce que le solvant est une huile de silicone.

6. Procédé selon les revendications 4 ou 5, caractérisé en ce que l'excès de solvant évaporé est récupéré et réutilisé.